# EUROPEAN PATENT SPECIFICATION

(11) **EP 1 492 553 B1**
(45) Date of publication and mention of the grant of the patent: **22.11.2006**
(21) Application number: 03743885.0
(22) Date of filing: 14.03.2003
(51) Int. Cl.: A61K 38/16, A61P 3/08

(54) **CG8327 AND SRM INVOLVED IN THE REGULATION OF ENERGY HOMEOSTASIS**
CG8327 UND SRM IN DER REGULATION VON ENERGIE-HOMEOSTASE
CG8327 ET SRM INTERVENANT DANS LA REGULATION DE L'HEMOSTASIE ENERGETIQUE

(30) Priority: 14.03.2002 EP 02005882; 15.03.2002 EP 02006012; 20.03.2002 EP 02006271; 25.03.2002 EP 02006810
(43) Date of publication of application: 05.01.2005
(73) Proprietor: DeveloGen Aktiengesellschaft, 37079 Göttingen (DE)
(72) Inventor: EULENBERG, Karsten, 37120 Bovendenn (DE); STEUERNAGEL, Arnd, 37085 Göttingen (DE); HÄDER, Thomas, 37073 Göttingen (DE); BRÖNNER, Günter, 37077 Göttingen (DE)
(74) Representative: Weiss, Wolfgang
(86) International application number: PCT/EP2003/002714
(87) International publication number: WO 2003/075945

(56) References cited:
- WO-A-02/12338
- WO-A-02/058623
- DATABASE BIOSIS [Online] BIOSCIENCES INFORMATION SERVICE, PHILADELPHIA, PA, US; 1982, ITO H ET AL: "ANTI TUMOR EFFECT OF DI CYCLO HEXYL AMMONIUM SULFATE A POTENT INHIBITOR OF SPERMIDINE SYNTHASE AGAINST P-388 LEUKEMIA" XP002261386 Database accession no. PREV198274055345 & CANCER LETTERS, vol. 15, no. 3, 1982, pages 229-236, ISSN: 0304-3835
- DATABASE WPI Section Ch, Week 200011 Derwent Publications Ltd., London, GB; Class B04, AN 2000-126652 XP002261387 & WO 99/67637 A (MERCK PATENT GMBH) 29 December 1999 (1999-12-29)
- DATABASE WPI Section Ch, Week 200217 Derwent Publications Ltd., London, GB; Class B04, AN 2002-130151 XP002261388 & WO 01/018210 A (GENENTECH INC) 15 March 2001 (2001-03-15)
- DATABASE WPI Section Ch, Week 200214 Derwent Publications Ltd., London, GB; Class B04, AN 2002-106464 XP002261389 & WO 01/096371 A (DEVELOGEN AG) 20 December 2001 (2001-12-20)
- DATABASE WPI Section Ch, Week 200017 Derwent Publications Ltd., London, GB; Class B04, AN 2000-195157 XP002261390 & WO 00/06087 A (TULARIK INC) 10 February 2000 (2000-02-10)
- DATABASE WPI Section Ch, Week 200163 Derwent Publications Ltd., London, GB; Class B04, AN 2001-565582 XP002261391 & WO 01/066706 A (HUMAN GENOME SCI INC) 13 September 2001 (2001-09-13)
- DATABASE WPI Section Ch, Week 200224 Derwent Publications Ltd., London, GB; Class B04, AN 2002-188506 XP002261392 & WO 02/05810 A (JOSLIN DIABETES CENT) 24 January 2002 (2002-01-24)

## Description

This invention relates to the use of nucleic acid sequences encoding Gadfly Accession Number CG8327, homologous proteins, and the polypeptides encoded thereby and to the use thereof in the manufacture of a medicament for the prevention, and treatment of obesity as well as related disorders such as eating disorder, cachexia, diabetes mellitus, hypertension, coronary heart disease, hypercholesterolemia, dyslipidemia,

There are several metabolic diseases of human and animal metabolism, eg., obesity and severe weight loss, that relate to energy imbalance where caloric intake versus energy expenditure is imbalanced. Obesity is one of the most prevalent metabolic disorders in the world. It is still a poorly, understood human disease that becomes more and more relevant for Western society. Obesity is defined as an excess of body fat, frequently resulting in a significant impairment of health. Cardiovascular risk factors like hypertension, high blood levels of triglycerides and fasting glucose as well as low blood levels of HDL cholesterol are often linked to obesity. This typical cluster of symptoms is commonly defined as "metabolic syndrome" (Reaven, 2002, Circulation 106(3): 286-288). Hyperlipidemia and elevation of free fatty acids correlate clearly with the metabolic syndrome, which is defined as the linkage between several diseases, including obesity and insulin resistance. This often occurs in the same patients and is a major risk factor for development of Type 2 diabetes and cardiovascular disease. It was suggested that the control of lipid levels and glucose levels is required to treat Type 2 Diabetes, heart disease, and other occurances of Metabolic Syndrome (see, for example, Santomauro A. T. et al., (1999) Diabetes, 48(9):1836-1841 and McCook, 2002, JAMA 288:2709-2716).

Human obesity is strongly influenced by environmental and genetic factors, whereby the environmental influence is often a hurdle for the identification of (human) obesity genes. Obesity is influenced by genetic, metabolic, biochemical, psychological, and behavioral factors. As such, it is a complex disorder that must be addressed on several fronts to achieve lasting positive clinical outcome.

Obesity is not to be considered as a single disorder but a heterogeneous group of conditions with (potential) multiple causes. Obesity is also characterized by elevated fasting plasma insulin and an exaggerated insulin response to oral glucose intake (Koltermann, J. Clin. Invest 65, 1980, 1272-1284) and a clear involvement of obesity in type 2 diabetes mellitus can be confirmed (Kopelman, Nature 404, 2000, 635-643).

Insulin amongst other hormones plays a key role in the regulation of the fuel metabolism. High blood glucose levels stimulate the secretion of insulin by pancreatic beta-cells. Insulin leads to the storage of glycogen and triglycerides and to the synthesis of proteins. The entry of glucose into muscles and adipose cells is stimulated by insulin. In patients who suffer from diabetes mellitus either the amount of insulin produced by the pancreatic islet cells is to low (Diabetes Type 1 or insulin dependent diabetes mellitus IDDM) or liver and muscle cells loose their ability to respond to normal blood insulin levels (insulin resistance). In the next stage pancreatic cells become unable to produce sufficient amounts of insulin (Diabetes Type II or non insulin dependent diabetes mellitus NIDDM).

Even if several candidate genes have been described which are supposed to influence the homeostatic system(s) that regulate body mass/weight, like leptin, VCPI, VCPL, or the peroxisome proliferator-activated receptor-gamma co-activator, the distinct molecular mechanisms and/or molecules influencing obesity or body weight/body mass regulations are not known.

Therefore, the technical problem underlying the present invention was to provide for means and methods for modulating (pathological) metabolic conditions influencing body-weight regulation and/or energy homeostatic circuits. The solution to said technical problem is achieved by providing the embodiments characterized in the claims.

Accordingly, the present invention relates to genes with novel functions in body-weight regulation, energy homeostasis, metabolism, and obesity. The present invention discloses a specific gene involved in the regulation of body-weight, energy homeostasis, metabolism, and obesity, and thus in disorders related thereto such as metabolic syndrome, eating disorder, cachexia, diabetes mellitus, hypertension, coronary heart disease, hypercholesterolemia, dyslipidemia, osteoarthritis, and gallstones. The present invention describes the human homologs of the Drosophila Gadfly Accession Number CG8327, gene as being involved in those conditions mentioned above.

Polyamines (putrescine, spermidine, and spermine) can modulate the functions of RNA, DNA, nucleotide triphosphates, proteins, and other acidic substances (Igarashi K. and Kashiwagi K., 2000, Biochem Biophys Res Commun 271(3):559-564). Polyamines stimulate the activity of glycogen synthase (casein) kinase-1 from bovine kidney and different rat tissues (Singh T.J., 1988, Arch Biochem Biophys 267(1):167-175). The biosynthesis of polyamines, which are universally essential for cellular functions, takes place from arginine and methionine and involves 4 distinct enzymes: spermidine synthase, ornithine decarboxylase, S-adenosyl-L-methionine decarboxylase, and spermine synthase (Janne J. et al., 1991, Ann Med 23(3):241-259). Wahlfors et al. (1990, DNA Cell Biol. 9:103-110) cloned a cDNA coding for the full-length subunit of human spermidine synthase. Myohanen et al. (1991, DNA Cell Biol. 10(6):467-474) isolated the human gene from a genomic library and mapped it to chromosome 1. A transgenic mouse line over-expresses the human spermidine synthase gene. The elevated spermidine synthase activity has no effect on tissue putrescine, spermidine or spermine levels (Kauppinen L. et al., 1993, Biochem J 293 (Pt 2):513-516).

WO02/12338 discloses the use of human spermidine synthase for the screening of pain-regulating substances.

WO02/058623, state of the art under Article 54(3) and (4) EPC only, discloses the use of an inhibitor of spermidine synthase to treat osteoarthritis.

Ito H. et al., 1982, Cancer letters, vol. 15, no. 3, 229-236, discloses the use of an inhibitor to spermidine synthase in the treatment of cancer.

So far, it has not been described that Gadfly Accession Number CG8327 encoded proteins and closely related proteins, particularly human protein spermidine synthase (SRM) is involved in the regulation of energy homeostasis and body-weight regulation and related disorders, and thus, no functions in metabolic diseases and other diseases as listed above have been discussed.

In this invention we demonstrate that the correct gene dose of Gadfly Accession Number CG8327 is essential for maintenance of energy homeostasis. A genetic screen was used to identify that mutation of Gadfly Accession Number CG8327 homologous genes cause obesity, reflected by a significant increase of triglyceride content, the major energy storage substance.

Polynucleotides encoding proteins with homologies to Gadfly Accession Number CG8327 are suitable to investigate diseases and disorders as described above. Further new compositions useful in diagnosis, treatment, and prognosis of diseases and disorders as described above are provided.

Before the present proteins, nucleotide sequences, and methods are described, it is understood that this invention is not limited to the particular methodology, protocols, cell lines, vectors, and reagents described as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. All publications mentioned herein are incorporated herein by reference for the purpose of describing and disclosing the cell lines, vectors, and methodologies that are reported in the publications which might be used in connection with the invention. Nothing herein is to be construed as an admission that the invention is not entitled to antedate such disclosure.

The present invention discloses that Gadfly Accession Number CG8327 homologous proteins are regulating the energy homeostasis and fat metabolism especially the metabolism and storage of triglycerides, and polynucleotides, which identify and encode the proteins disclosed in this invention. The invention also relates to vectors, host cells, antibodies, and recombinant methods for producing the polypeptides and polynucleotides of the invention. The invention also relates to the use of these sequences in the diagnosis, study, prevention, and treatment of diseases and disorders, for example, but not limited to, metabolic diseases such as obesity as well as related disorders such as metabolic syndrome, eating disorder, cachexia, diabetes mellitus, hypertension, coronary heart disease, hypercholesterolemia, dyslipidemia, osteoarthritis, and gallstones.

The term "polynucleotide comprising the nucleotide sequence as shown in GenBank Accession number" relates to the expressible gene of the nucleotide sequences deposited under the corresponding GenBank Accession number. The term "GenBank Accession Number" relates to NCBI GenBank database entries (Ref.: Benson et al., (2000) Nucleic Acids Res. 28:15-18).

Gadfly Accession Number CG8327 homologous proteins and nucleic acid molecules coding therefore are obtainable from insect or vertebrate species, e.g. mammals or birds. Particularly preferred are homologous nucleic acids, particularly nucleic acid encoding a human spermidine synthase (SRM)

The invention particularly relates to a nucleic acid molecule encoding a polypeptide contributing to regulating the energy homeostasis and the metabolism of triglycerides, wherein said nucleic acid molecule comprises
(a) the nucleotide sequence of or a nucleotide sequence encoding (i) Gadfly Accession Number CG8327, human spermidine synthase (SEQ ID NO: 1; GenBank Accession Number NM_003132) and/or a sequence complementary thereto,
(b) a nucleotide sequence which hybridizes at 50°C in a solution containing 1 x SSC and 0.1 % SDS to a sequence of (a),
(c) a sequence corresponding to the sequences of (a) or (b) within the degeneration of the genetic code,
(d) a sequence which encodes a polypeptide which is at least 85%, preferably at least 90%, more preferably at least 95%, more preferably at least 98% and up to 99,6% identical to the amino acid sequences of Gadfly Accession Number CG8327
(e) a sequence encoding a Gadfly Accession Number CG8327 and/or homologous protein, particularly human spermidine synthase (SEQ ID NO: 2; GenBank Accession Number NP_003123)
(f) a sequence which differs from the nucleic acid molecule of (a) to (d) by mutation and wherein said mutation causes an alteration, deletion, duplication and/or premature stop in the encoded polypeptide or
(g) a partial sequence of any of the nucleotide sequences of (a) to (e) having a length of at least 15 bases, preferably at least 20 bases, more preferably at least 25 bases and most preferably at least 50 bases.

The invention is based on the finding that Gadfly Accession Number CG8327, and/or homologous protein (herein referred to as SRM) and the polynucleotides encoding these, are involved in the regulation of triglyceride storage and therefore energy homeostasis. The invention describes the use of these polypeptides, polynucleotides and effectors thereof for the diagnosis, study, prevention, or treatment of diseases and disorders related thereto, including metabolic diseases such as obesity as well as related disorders such as metabolic syndrome, eating disorder, cachexia, diabetes mellitus, hypertension, coronary heart disease, hypercholesterolemia, dyslipidemia, osteoarthritis, or gallstones.

Accordingly, the present invention relates to genes with novel functions in body-weight regulation, energy homeostasis, metabolism, and obesity, fragments of said genes, polypeptides encoded by said genes or fragments thereof, and antibodies, biologically active nucleic acids, such as antisense molecules, RNAi molecules or ribozymes or aptamers.

The ability to manipulate and screen the genomes of model organisms such as the fly Drosophila melanogaster provides a powerful tool to analyze biological and biochemical processes that have direct relevance to more complex vertebrate organisms due to significant evolutionary conservation of genes, cellular processes, and pathways (see, for example, Adams M. D. et al., (2000) Science 287: 2185-2195). Identification of novel gene functions in model organisms can directly contribute to the elucidation of correlative pathways in mammals (humans) and of methods of modulating them. A correlation between a pathology model (such as changes in triglyceride levels as indication for metabolic syndrome including obesity) and the modified expression of a fly gene can identify the association of the human ortholog with the particular human disease.

A forward genetic screen can be performed in fly displaying a mutant phenotype due to misexpression of a known gene (see, Johnston Nat Rev Genet 3: 176-188 (2002); Rorth P., (1996) Proc Natl Acad Sci U S A 93: 12418-12422). Triglycerides are the most efficient storage for energy in cells. Obese people mainly show a significant increase in the content of triglycerides. In this invention, we have used a genetic screen to identify mutations of genes encoding the proteins of the invention and homologous genes that cause changes in the body weight which is reflected by a significant change of triglyceride levels. In order to isolate genes with a function in energy homeostasis, several thousand proprietary and publicly available EP-lines were tested for their triglyceride content after a prolonged feeding period (see Examples for more detail). Lines with significantly changed triglyceride content were selected as positive candidates for further analysis. The change of triglyceride content due to the loss of a gene function suggests gene activities in energy homeostasis in a dose dependent manner that control the amount of energy stored as triglycerides.

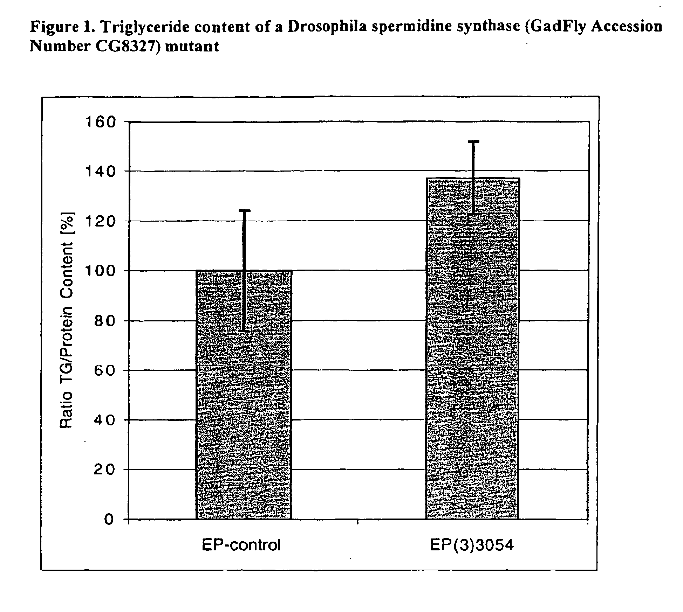

In one embodiment, flies homozygous for the integration of vectors for Drosophila lines EP(3)3054, HD-EP(3)31068, HD-EP(3)31149, EP(3)3322, EP(2)2162, EP(3)0650, HD-EP(3)31393, and HD-EP(3)32007 were analyzed in an assay measuring the triglyceride contents of these flies, illustrated in more detail in the EXAMPLES section of the invention. The results of the triglyceride content analysis are shown in Figure 1.

The increase of triglyceride content due to the loss of a gene function suggests gene activities in energy homeostasis in a dose dependent manner that controls the amount of energy stored as triglycerides.

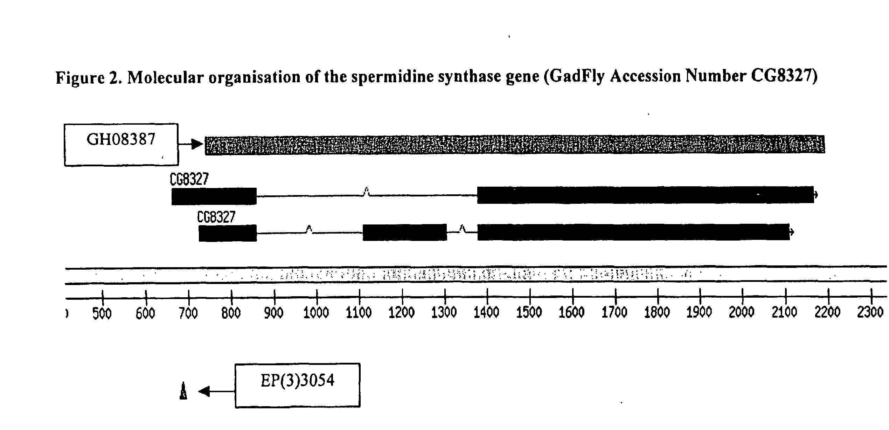

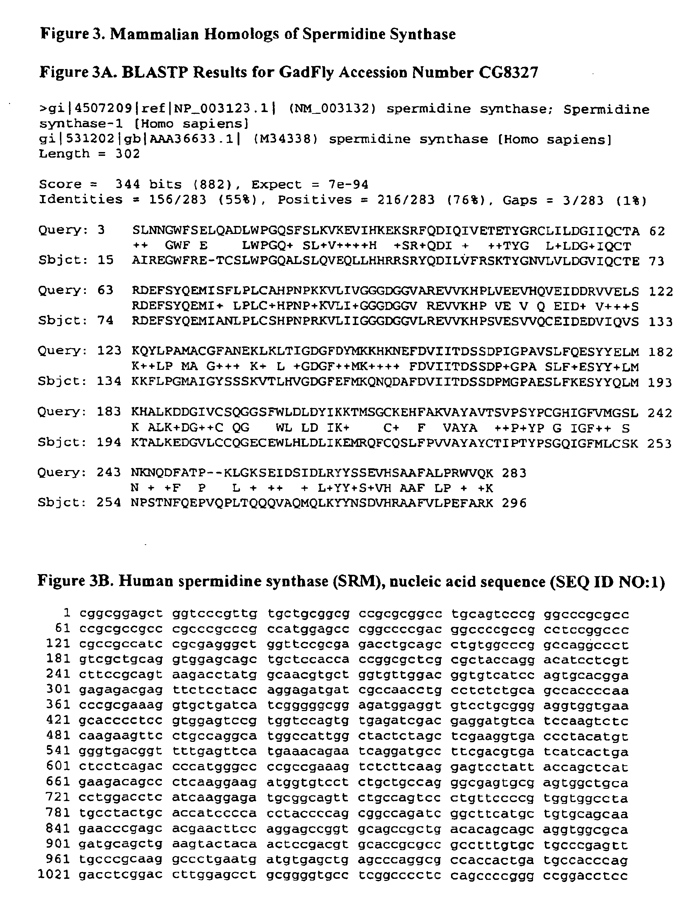

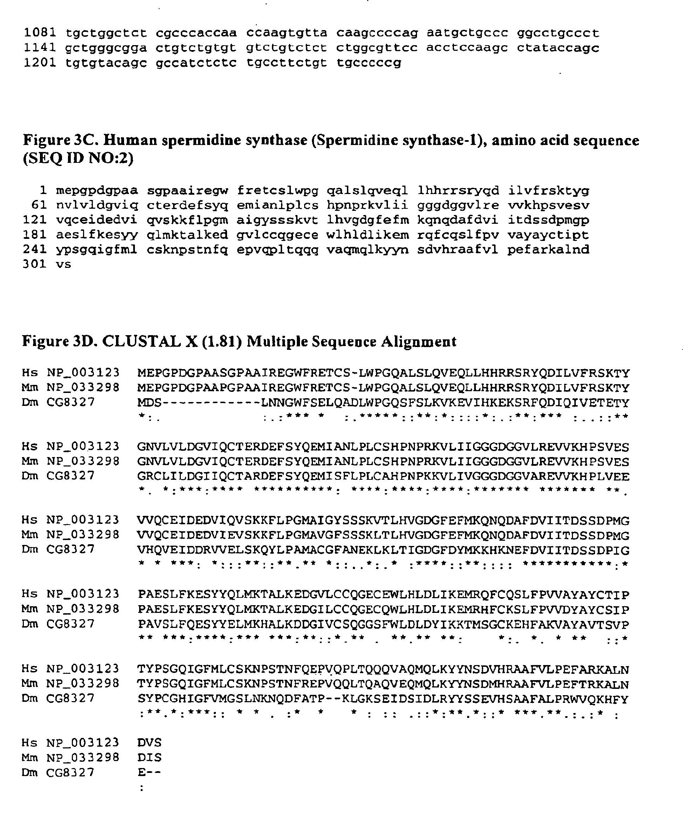

Nucleic acids encoding the proteins of the present invention were identified using a plasmid-rescue technique. Genomic DNA sequences were isolated that are localized adjacent to the EP vector (herein EP(3)3054, HD-EP(3)31068, HD-EP(3)31149, EP(3)3322, EP(2)2162, EP(3)0650, HD-EP(3)31393, and HD-EP(3)32007) integration. Using those isolated genomic sequences public databases like Berkeley Drosophila Genome Project (GadFly; see also FlyBase (1999) Nucleic Acids Research 27:85-88) were screened thereby identifying the integration sites of the vectors, and the corresponding genes, described in more detail in the EXAMPLES section. The molecular organization of the genes is shown in Figure 2. The Drosophila genes and proteins encoded thereby with functions in the regulation of triglyceride metabolism were further analysed in publicly available sequence databases (see EXAMPLES for more detail) and mammalian homologs were identified (see Figure 3). Comparisons (Clustal analysis) between the proteins of different species (human, mouse and Drosophila) were conducted (see Figure 3). Based upon homology, the Drosophila proteins of the invention and each homologous protein or peptide may share at least some activity. No functional data described the regulation of body weight control and related metabolic diseases such as obesity and diabetes are available in the prior art for the genes of the invention.

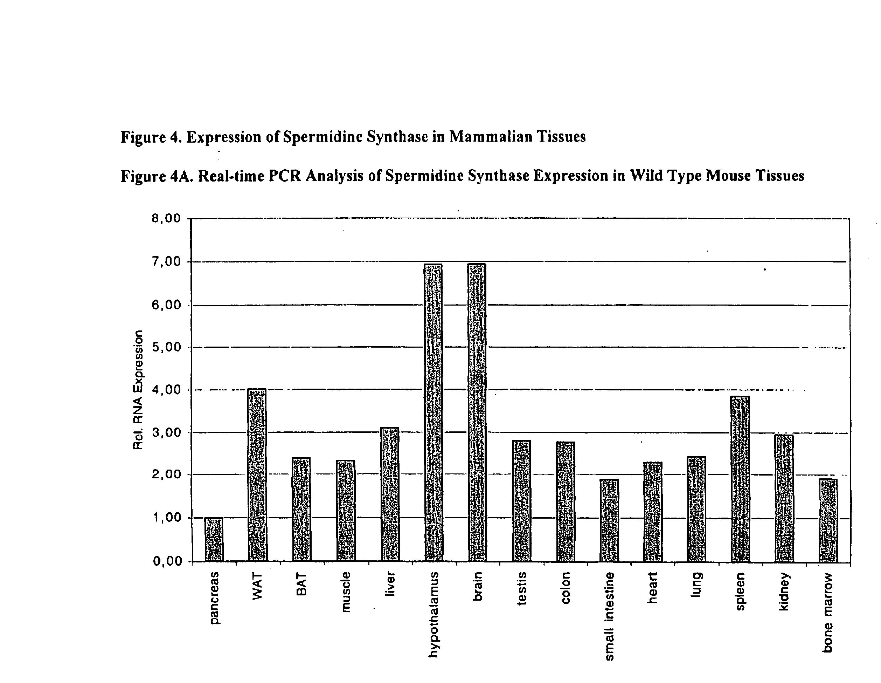

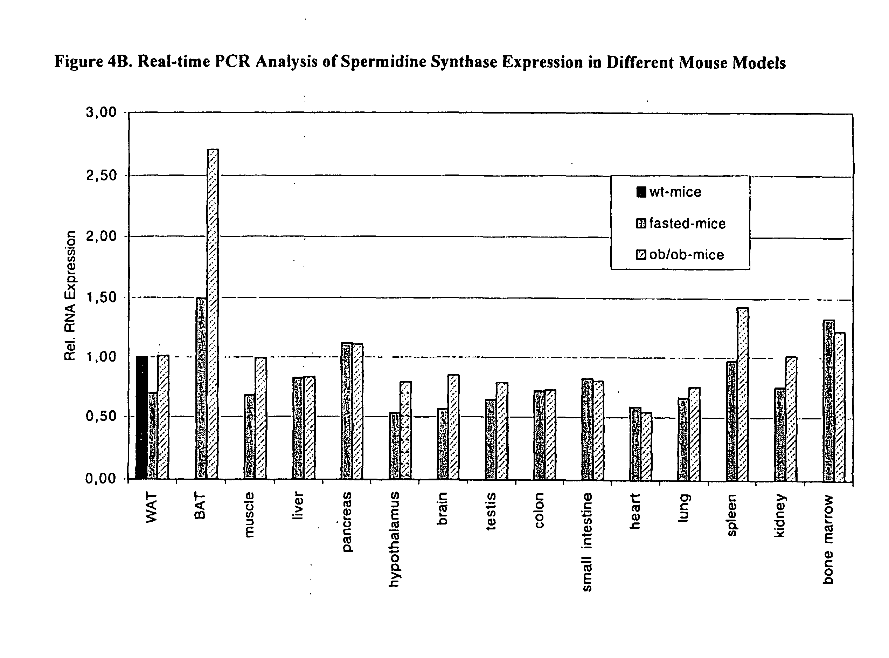

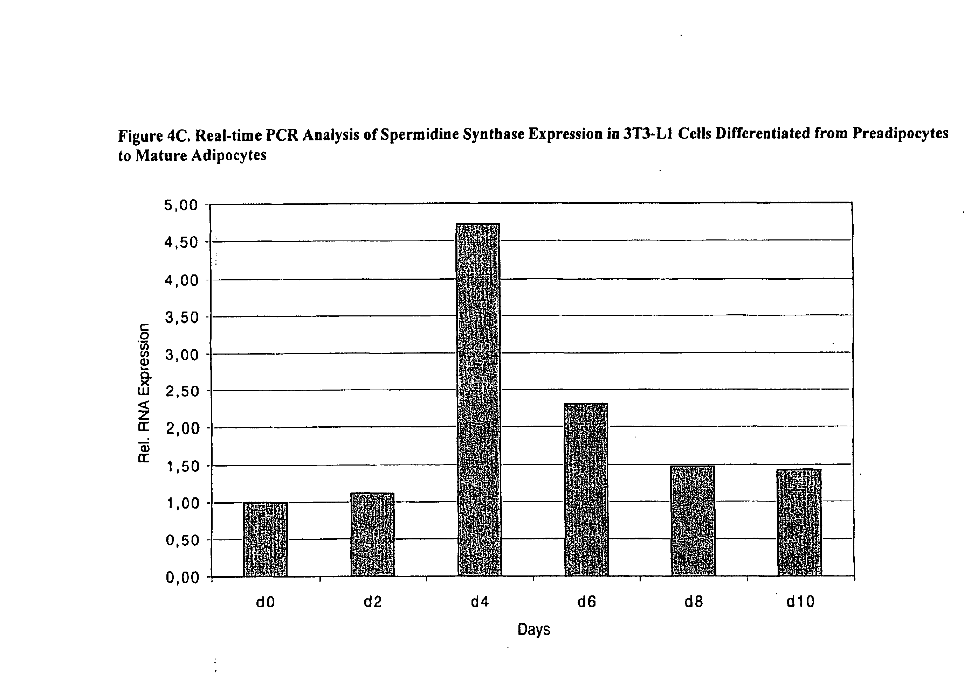

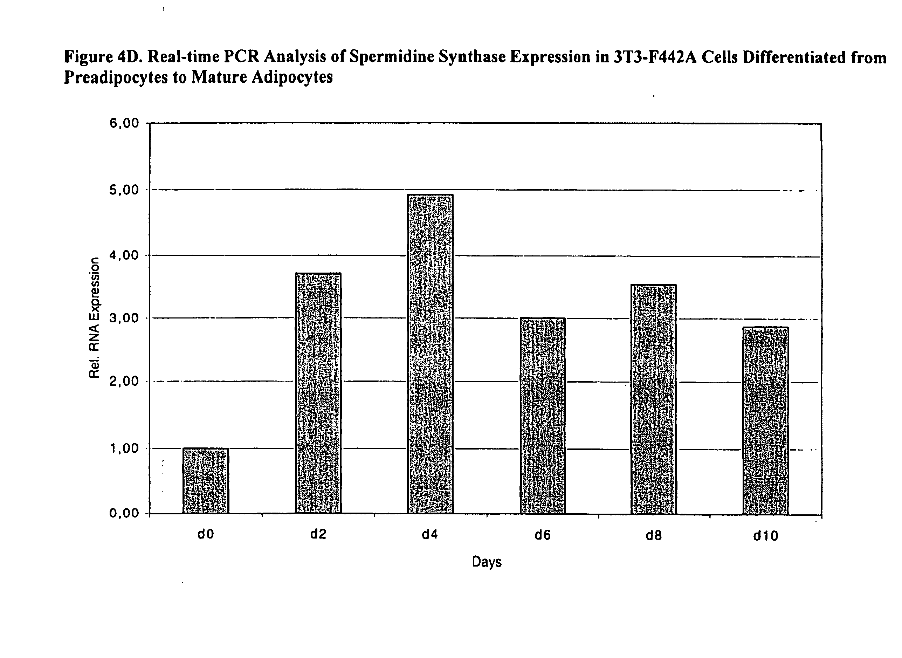

The function of the mammalian homologs in energy homeostasis was further validated in this invention by analyzing the expression of the transcripts in different tissues and by analyzing the role in adipocyte differentiation (see Figure 4). Expression profiling studies (see Examples for more detail) confirm the particular relevance of the proteins of the invention as regulators of enery metabolism in mammals. Further, we show that the proteins of the invention are regulated by fasting or by genetically induced obesity. In this invention, we used mouse models of insulin resistance and/or diabetes, such as mice carrying gene knockouts in the leptin pathway (for example, ob (leptin) or db (leptin receptor) mice) to study the expression of the proteins of the invention. Such mice develop typical symptoms of diabetes, show hepatic lipid accumulation, and frequently have increased plasma lipid levels (see Bruning et al, 1998, Mol. Cell. 2:449-569). In addition, we show in this invention that the mRNAs of the proteins of the invention are regulated during adipocyte differentiation in vitro (see EXAMPLES for more detail), suggesting a role as modulator of adipocyte lipid accumulation. Thus, we conclude that the protein of the invention (or variants thereof) have a function in the metabolism of mature mammalian adipocytes.

The present invention further describes polypeptides comprising the amino acid sequences of SRM and homologous proteins. Based upon homology, the proteins of the invention and each homologous protein or peptide may share at least some activity. No functional data described the regulation of body weight control and related metabolic diseases such as obesity are available in the prior art for the genes of the invention.

The invention also encompasses polynucleotides that encode a protein of the invention or homologous proteins. Accordingly, any nucleic acid sequence, which encodes the amino acid sequences of a protein of the invention or homologous proteins, can be used to generate recombinant molecules that express a protein of the invention or homologous proteins. It will be appreciated by those skilled in the art that as a result of the degeneracy of the genetic code, a multitude of nucleotide sequences encoding the proteins of the invention or homologous proteins, some bearing minimal homology to the nucleotide sequences of any known and naturally occurring gene, may be produced. Thus, the invention contemplates each and every possible variation of nucleotide sequence that can be made by selecting combinations based on possible codon choices.

Also encompassed by the invention are polynucleotide sequences that are capable of hybridizing to the claimed nucleotide sequences, and in particular, those of the polynucleotide encoding a protein of the invention, under various conditions of stringency. Hybridization conditions are based on the melting temperature (Tm) of the nucleic acid binding complex or probe, as taught in Wahl, G. M. and S. L. Berger (1987: Methods Enzymol. 152:399-407) and Kimmel, A. R. (1987; Methods Enzymol. 152:507-511), and may be used at a defined stringency. Preferably, hybridization under stringent conditions means that after washing for 1 h with 1 x SSC and 0.1 % SDS at 50°C, preferably at 55°C, more preferably at 62°C and most preferably at 68°C, particularly for 1 h in 0.2 x SSC and 0.1% SDS at 50°C, preferably at 55°C, more preferably at 62°C and most preferably at 68°C, a positive hybridization signal is observed. Altered nucleic acid sequences encoding a protein of the invention or homologous proteins which are encompassed by the invention include deletions, insertions, or substitutions of different nucleotides resulting in a polynucleotide that encodes the same or a functionally equivalent protein.

The encoded proteins may also contain deletions, insertions, or substitutions of amino acid residues, which produce a silent change and result in functionally equivalent proteins. Deliberate amino acid substitutions may be made on the basis of similarity in polarity, charge, solubility, hydrophobicity, hydrophilicity, and/or the amphipathic nature of the residues as long as the biological activity of the protein is retained. Furthermore, the invention relates to peptide fragments of the proteins or derivates of such peptides such as cyclic peptides, retro-inverso peptides or peptide mimetics having a length of at least 4, preferably at least 6 and up to 50 amino acids.

Also included within the scope of the present invention are alleles of the genes encoding a protein of the invention or homologous proteins. As used herein, an 'allele' or 'allelic sequence' is an alternative form of the gene, which may result from at least one mutation in the nucleic acid sequence. Alleles may result in altered mRNAs or polypeptides whose structures or function may or may not be altered. Any given gene may have none, one, or many allelic forms. Common mutational changes, which give rise to alleles, are generally ascribed to natural deletions, additions, or substitutions of nucleotides. Each of these types of changes may occur alone, or in combination with the others, one or more times in a given sequence.

The nucleic acid sequences encoding a protein of the invention or homologous proteins may be extended utilizing a partial nucleotide sequence and employing various methods known in the art to detect upstream sequences such as promoters and regulatory elements. For example, one method which may be employed, "restriction-site" PCR, uses universal primers to retrieve unknown sequence adjacent to a known locus (Sarkar, G. (1993) PCR Methods Applic. 2:318-322). Inverse PCR may also be used to amplify or extend sequences using divergent primers based on a known region (Triglia, T. et al. (1988) Nucleic Acids Res. 16:8186). Another method which may be used is capture PCR which involves PCR amplification of DNA fragments adjacent to a known sequence in human and yeast artificial chromosome DNA (Lagerstrom, M. et al. (PCR Methods Applic. 1:111-119). Another method which may be used to retrieve unknown sequences is that of Parker, J. D. et al. (1991; Nucleic Acids Res. 19:3055-3060). Additionally, one may use PCR, nested primers, and PROMOTERFINDER libraries to walk in genomic DNA (Clontech, Palo Alto, Calif.). This process avoids the need to screen libraries and is useful in finding intron/exon junctions.

In order to express a biologically active protein, the nucleotide sequences encoding the protein or functional equivalents may be inserted into appropriate expression vectors, i.e. a vector, which contains the necessary elements for the transcription and translation of the inserted coding sequence. Methods, which are well known to those skilled in the art, may be used to construct expression vectors containing sequences encoding the proteins and appropriate transcriptional and translational control elements. These methods include in vitro recombinant DNA techniques, synthetic techniques, and in vivo genetic recombination. Such techniques are described in Sambrook, J. et al. (1989) Molecular Cloning, A Laboratory Manual, Cold Spring Harbor Press, Plainview, N.Y., and Ausubel, F. M. et al. (1989) Current Protocols in Molecular Biology, John Wiley & Sons, New York, N.Y.

A variety of expression vector/host systems may be utilized to contain and express sequences encoding a protein of the invention or homologous proteins. These include, but are not limited to, micro-organisms such as bacteria transformed with recombinant bacteriophage, plasmid, or cosmid DNA expression vectors; yeast transformed with yeast expression vectors; insect cell systems infected with virus expression vectors (e,g., baculovirus); plant cell systems transformed with virus expression vectors (e.g., cauliflower mosaic virus, CaMV; tobacco mosaic virus, TMV) or with bacterial expression vectors (e.g., Ti or PBR322 plasmids); or animal cell systems. The "control elements" or "regulatory sequences" are those non-translated regions of the vector-enhancers, promoters, 5' and 3' untranslated regions which interact with host cellular proteins to carry out transcription and translation. Such elements may vary in their strength and specificity. Depending on the vector system and host utilized, any number of suitable transcription and translation elements, including constitutive and inducible promoters, may be used.

The presence of polynucleotide sequences encoding a protein of the invention or homologous proteins can be detected by DNA-DNA or DNA-RNA hybridization and/or amplification using probes or portions or fragments of polynucleotides encoding the protein. Nucleic acid amplification based assays involve the use of oligonucleotides or oligomers based on the sequences specific for a protein of the invention and nucleic acids to detect transformants containing DNA or RNA encoding the proteins. As used herein 'oligonucleotides' or 'oligomers' refer to a nucleic acid sequence of at least about 10 nucleotides and as many as about 60 nucleotides, preferably about 15 to 30 nucleotides, and more preferably about 20-25 nucleotides, which can be used as a probe or amplimer.

A variety of protocols for detecting and measuring the expression of the proteins of the invention or homologous proteins, using either polyclonal or monoclonal antibodies specific for the protein are known in the art. Examples include enzyme-linked immunosorbent assay (ELISA), radioimmunoassay (RIA), and fluorescence activated cell sorting (FACS). A two-site, monoclonal-based immunoassay utilizing monoclonal antibodies reactive to two non-interfering epitopes on the protein is preferred, but a competitive binding assay may be employed. These and other assays are described, among other places, in Hampton, R. et al. (1990; Serological Methods, a Laboratory Manual, APS Press, St Paul, Minn.) and Maddox, D. E. et al. (1983; J. Exp. Med. 158:1211-1216).

A wide variety of labels and conjugation techniques are known by those skilled in the art and may be used in various nucleic acid and amino acid assays. Means for producing labeled hybridization or PCR probes for detecting sequences related to polynucleotides encoding a protein of the invention or homologous proteins include oligo-labeling, nick translation, end-labeling or PCR amplification using a labeled nucleotide.

Alternatively, the sequences encoding a protein of the invention or homologous proteins, or any portions thereof may be cloned into a vector for the production of an mRNA probe. Such vectors are known in the art, are commercially available, and may be used to synthesize RNA probes in vitro by addition of an appropriate RNA polymerase such as T7, T3, or SP6 and labeled nucleotides. These procedures may be conducted using a variety of commercially available kits (Pharmacia & Upjohn, (Kalamazoo, Mich.); Promega (Madison Wis.); and U.S. Biochemical Corp., (Cleveland, Ohio).

Suitable reporter molecules or labels, which may be used, include radionuclides, enzymes, fluorescent, chemiluminescent, or chromogenic agents as well as substrates, co-factors, inhibitors, magnetic particles, and the like.

Host cells transformed with nucleotide sequences encoding a protein of the invention or homologous proteins may be cultured under conditions suitable for the expression and recovery of the protein from cell culture. The protein produced by a recombinant cell may be secreted or contained intracellularly depending on the sequence and/or the vector used. As will be understood by those of skill in the art, expression vectors containing polynucleotides which encode the protein may be designed to contain signal sequences, which direct secretion of the protein through a prokaryotic or eukaryotic cell membrane. Other recombinant constructions may be used to join sequences encoding the desired protein to a nucleotide sequence encoding a polypeptide domain, which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAG extension/affinity purification system (lmmunex Corp., Seattle, Wash.)

### Diagnostics and Therapeutics

The data disclosed in this invention show that the nucleic acids and proteins of the invention and effector molecules thereof are useful in therapeutic applications implicated, for example but not limited to, in metabolic disorders such as obesity as well as related disorders such as metabolic syndrome, eating disorder, cachexia, diabetes mellitus, hypertension, coronary heart disease, hypercholesterolemia, dyslipidemia, osteoarthritis, and gallstones. Hence, therapeutic uses for the nucleic acids and proteins of the present invention, and homologous nucleic acids and proteins of the invention are, for example but not limited to, the following: (i) protein therapeutic, (ii) antibody target (therapeutic, diagnostic, drug targeting/cytotoxic antibody), (iii) gene therapy (gene delivery/gene ablation), and (iv) tissue regeneration in vitro and in vivo (regeneration for all these tissues and cell types composing these tissues and cell types derived from these tissues).

The nucleic acids and proteins of the invention are useful in therapeutic applications implicated in various applications as described below. For example, but not limited to, cDNAs encoding the proteins of the invention and particularly their human homologues may be useful in gene therapy, and the proteins of the invention and particularly their human homologues may be useful when administered to a subject in need thereof. By way of non-limiting example, the compositions of the present invention will have efficacy for treatment of patients suffering from, for example, but not limited to, in metabolic disorders as described above.

For example, in one aspect, antibodies which are specific for a protein of the invention or homologous proteins may be used directly as an antagonist, or indirectly as a targeting or delivery mechanism for bringing a pharmaceutical agent to cells or tissue which express the protein. The antibodies may be generated using methods that are well known in the art. Such antibodies may include, but are not limited to, polyclonal, monoclonal, chimerical, single chain, Fab fragments, and fragments produced by a Fab expression library. Neutralising antibodies, (i.e., those which inhibit dimer formation) are especially preferred for therapeutic use.

For the production of antibodies, various hosts including goats, rabbits, rats, mice, humans, and others, may be immunized by injection with a protein of the invention or any fragment or oligopeptide thereof which has immunogenic properties. Depending on the host species, various adjuvants may be used to increase immunological response. It is preferred that the peptides, fragments, or oligopeptides used to induce antibodies have an amino acid sequence consisting of at least five amino acids, and more preferably at least 10 amino acids.

Monoclonal antibodies may be prepared using any technique that provides for the production of antibody molecules by continuous cell lines in culture. These include, but are not limited to, the hybridoma technique, the human B-cell hybridoma technique, and the EBV-hybridoma technique (Köhler, G. et al. (1975) Nature 256:495-497; Kozbor, D. et al. (1985) J. Immunol. Methods 81:31-42; Cote, R. J. et al. Proc. Natl. Acad. Sci. 80:2026-2030; Cole, S. P. et al. (1984) Mol. Cell Biol. 62:109-120).

In addition, techniques developed for the production of "chimeric antibodies", the splicing of mouse antibody genes to human antibody genes to obtain a molecule with appropriate antigen specificity and biological activity can be used (Morrison, S. L. et al. (1984) Proc. Natl. Acad. Sci. 81:6851-6855; Neuberger, M. S. et al. (1984) Nature 312:604-608; Takeda, S. et al. (1985). Nature 314:452-454). Alternatively, techniques described for the production of single chain antibodies may be adapted, using methods known in the art, to produce specific single chain antibodies. Antibodies with related specificity, but of distinct idiotypic composition, may be generated by chain shuffling from random combinatorial immunoglobulin libraries (Burton, D. R. (1991) Proc. Natl. Acad. Sci. 88:11120-3). Antibodies may also be produced by inducing in vivo production in the lymphocyte population or by screening recombinant immunoglobulin libraries or panels of highly specific binding reagents as disclosed in the literature (Orlandi, R. et al. (1989) Proc. Natl. Acad. Sci. 86:3833-3837; Winter, G. et al. (1991) Nature 349:293-299).

Antibody fragments, which contain specific binding sites for the proteins, may also be generated. For example, such fragments include, but are not limited to, the F(ab')₂ fragments which can be produced by Pepsin digestion of the antibody molecule and the Fab fragments which can be generated by reducing the disulfide bridges of F(ab')₂ fragments. Alternatively, Fab expression libraries may be constructed to allow rapid and easy identification of monoclonal Fab fragments with the desired specificity (Huse, W. D. et al. (1989) Science 254:1275-1281).

Various immunoassays may be used for screening to identify antibodies having the desired specificity. Numerous protocols for competitive binding and immunoradiometric assays using either polyclonal or monoclonal antibodies with established specificities are well known in the art. Such immunoassays typically involve the measurement of complex formation between a protein of the invention or homologous proteins and its specific antibody. A two-site, monoclonal-based immunoassay utilising monoclonal antibodies reactive to two non-interfering protein epitopes are preferred, but a competitive binding assay may also be employed (Maddox, supra).

In another embodiment of the invention, the polynucleotides or fragments thereof, or nucleic acid effector molecules such as antisense molecules, aptamers, RNAi molecules or ribozymes may be used for therapeutic purposes. In one aspect, aptamers, i.e. nucleic acid molecules, which are capable of binding to a protein of the invention and modulating its activity, may be generated by a screening and selection procedure involving the use of combinatorial nucleic acid libraries.

In a further aspect, antisense molecules to the polynucleotide may be used in situations in which it would be desirable to block the transcription of the mRNA. In particular, cells may be transformed with sequences complementary to polynucleotides encoding the proteins. Thus, antisense molecules may be used to modulate protein activity, or to achieve regulation of gene function. Such technology is now well know in the art, and sense or antisense oligomers or larger fragments, can be designed from various locations along the coding or control regions of sequences encoding the proteins. Expression vectors derived from retroviruses, adenoviruses, herpes or vaccinia viruses, or from various bacterial plasmids may be used for delivery of nucleotide sequences to the targeted organ, tissue or cell population. Methods, which are well known to those skilled in the art, can be used to construct recombinant vectors, which will express antisense molecules complementary to the polynucleotides of the genes encoding the proteins. These techniques are described both in Sambrook et al. (supra) and in Ausubel et al. (supra). Genes encoding a protein of the invention or homologous proteins can be turned off by transforming a cell or tissue with expression vectors which express high levels of polynucleotide or fragment thereof which encodes the protein. Such constructs may be used to introduce untranslatable sense or antisense sequences into a cell. Even in the absence of integration into the DNA, such vectors may continue to transcribe RNA molecules until they are disabled by endogenous nucleases. Transient expression may last for a month or more with a non-replicating vector and even longer if appropriate replication elements are part of the vector system.

As mentioned above, modifications of gene expression can be obtained by designing antisense molecules, e.g. DNA, RNA, or PNA, nucleic acid analogues such as to the control regions of the genes, i.e. the promoters, enhancers, and introns. Oligonucleotides derived from the transcription initiation site, e.g., between positions -10 and + 10 from the start site, are preferred. Similarly, inhibition can be achieved using "triple helix" base-pairing methodology. Triple helix pairing is useful because it cause inhibition of the ability of the double helix to open sufficiently for the binding of polymerases, transcription factors, or regulatory molecules. Recent therapeutic advances using triplex DNA have been described in the literature (Gee, J. E. et al. (1994) In; Huber, B. E. and B. I. Carr, Molecular and Immunologic Approaches, Futura Publishing Co., Mt. Kisco, N.Y.). The antisense molecules may also be designed to block translation of mRNA by preventing the transcript from binding to ribosomes.

Ribozymes, enzymatic RNA molecules, may also be used to catalyze the specific cleavage of RNA. The mechanism of ribozyme action involves sequence-specific hybridization of the ribozyme molecule to complementary target RNA, followed by endonucleolytic cleavage. Examples, which may be used, include engineered hammerhead motif ribozyme molecules that can be specifically and efficiently catalyze endonucleolytic cleavage of sequences encoding the proteins. Specific ribozyme cleavage sites within any potential RNA target are initially identified by scanning the target molecule for ribozyme cleavage sites which include the following sequences: GUA, GUU, and GUC. Once identified, short RNA sequences of between 15 and 20 ribonucleotides corresponding to the region of the target gene containing the cleavage site may be evaluated for secondary structural features which may render the oligonucleotide inoperable. The suitability of candidate targets may also be evaluated by testing accessibility to hybridization with complementary oligonucleotides using ribonuclease protection assays.

Nucleic acid effector molecules, e.g. antisense molecules and ribozymes of the invention may be prepared by any method known in the art for the synthesis of nucleic acid molecules. These include techniques for chemically synthesizing oligonucleotides such as solid phase phosphoramidite chemical synthesis. Alternatively, RNA molecules may be generated by in vitro and in vivo transcription of DNA sequences encoding the proteins. Such DNA sequences may be incorporated into a variety of vectors with suitable RNA polymerase promoters such as T7 or SP6. Alternatively, these cDNA constructs that synthesize antisense RNA constitutively or inducibly can be introduced into cell lines, cells, or tissues. RNA molecules may be modified to increase intracellular stability and half-life. Possible modifications include, but are not limited to, the addition of flanking sequences at the 5' and/or 3' ends of the molecule or the use of phosphorothioate or 2' O-methyl rather than phosphodiesterase linkages within the backbone of the molecule. This concept is inherent in the production of PNAs and can be extended in all of these molecules by the inclusion of non-traditional bases such as inosine, queosine, and wybutosine, as well as acetyl-, methyl-, thio-, and similarly modified forms of adenine, cytidine, guanine, thymine, and uridine which are not as easily recognized by endogenous endonucleases.

Many methods for introducing vectors into cells or tissues are available and equally suitable for use in vivo, in vitro, and ex vivo. For ex vivo therapy, vectors may be introduced into stem cells taken from the patient and clonally propagated for autologous transplant back into that same patient. Delivery by transfection and by liposome injections may be achieved using methods, which are well known in the art. Any of the therapeutic methods described above may be applied to any suitable subject including, for example, mammals such as dogs, cats, cows, horses, rabbits, monkeys, and most preferably, humans.

An additional embodiment of the invention relates to the administration of a pharmaceutical composition, in conjunction with a pharmaceutically acceptable carrier, for any of the therapeutic effects discussed above. Such pharmaceutical compositions may comprise a protein of the invention or homologous proteins, antibodies to a protein of the invention or homologous proteins, mimetics, agonists, antagonists, or inhibitors of a protein of the invention or homologous proteins. The compositions may be administered alone or in combination with at least one other agent, such as stabilizing compound, which may be administered in any sterile, biocompatible pharmaceutical carrier, including, but not limited to, saline, buffered saline, dextrose, and water. The compositions may be administered to a patient alone, or in combination with other agents, drugs or hormones. The pharmaceutical compositions utilized in this invention may be administered by any number of routes including, but not limited to, oral, intravenous, intramuscular, intra-arterial, intramedullary, intrathecal, intraventricular, transdermal, subcutaneous, intraperitoneal, intranasal, enteral, topical, sublingual, or rectal means.

In addition to the active ingredients, these pharmaceutical compositions may contain suitable pharmaceutically-acceptable carriers comprising excipients and auxiliaries, which facilitate processing of the active compounds into preparations which, can be used pharmaceutically. Further details on techniques for formulation and administration may be found in the latest edition of Remington's Pharmaceutical Sciences (Maack Publishing Co., Easton, Pa.).

The pharmaceutical compositions of the present invention may be manufactured in a manner that is known in the art, e.g., by means of conventional mixing, dissolving, granulating, dragee-making, levigating, emulsifying, encapsulating, entrapping, or lyophilizing processes. After pharmaceutical compositions have been prepared, they can be placed in an appropriate container and labeled for treatment of an indicated condition. For administration of a protein of the invention or homologous proteins, such labeling would include amount, frequency, and method of administration.

Pharmaceutical compositions suitable for use in the invention include compositions wherein the active ingredients are contained in an effective amount to achieve the intended purpose. The determination of an effective dose is well within the capability of those skilled in the art. For any compounds, the therapeutically effective does can be estimated initially either in cell culture assays, e.g., of preadipocyte cell lines, or in animal models, usually mice, rabbits, dogs, or pigs. The animal model may also be used to determine the appropriate concentration range and route of administration. Such information can then be used to determine useful doses and routes for administration in humans. A therapeutically effective dose refers to that amount of active ingredient, for example a protein of the invention or homologous proteins, fragments thereof, antibodies of a protein of the invention or homologous proteins etc., which is effective for the treatment of a specific condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. Pharmaceutical compositions, which exhibit large therapeutic indices, are preferred. The data obtained from cell culture assays and animal studies is used in formulating a range of dosage for human use. The dosage contained in such compositions is preferably within a range of circulating concentrations that include the ED50 with little or no toxicity. The dosage varies within this range depending upon the dosage from employed, sensitivity of the patient, and the route of administration. The exact dosage will be determined by the practitioner, in light of factors related to the subject that requires treatment. Dosage and administration are adjusted to provide sufficient levels of the active moiety or to maintain the desired effect.

Factors, which may be taken into account, include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half-life and clearance rate of the particular formulation. Normal dosage amounts may vary from 0.1 to 100,000 micrograms, up to a total dose of about 1 g, depending upon the route of administration. Guidance as to particular dosages and methods of delivery is provided in the literature and generally available to practitioners in the art. Those skilled in the art employ different formulations for nucleotides than for proteins or their inhibitors. Similarly, delivery of polynucleotides or polypeptides will be specific to particular cells, conditions, locations, etc.

The nucleic acids encoding the proteins of the invention can be used to generate transgenic cell lines and animals. These transgenic non-human animals are useful in the study of the function and regulation of the proteins of the invention in vivo. Transgenic animals, particularly mammalian transgenic animals, can serve as a model system for the investigation of many developmental and cellular processes common to humans. A variety of non-human models of metabolic disorders can be used to test modulators of the protein of the invention. Misexpression (for example, overexpression or lack of expression) of the protein of the invention, particular feeding conditions, and/or administration of biologically active compounts can create models of metablic disorders.

In one embodiment of the invention, such assays use mouse models of insulin resistance and/or diabetes, such as mice carrying gene knockouts in the leptin pathway (for example, ob.(leptin) or db (leptin receptor) mice). Such mice develop typical symptoms of diabetes , show hepatic lipid accumulation and frequently have increased plasma lipid levels (see Bruning et al, 1998, Mol. Cell. 2:449-569). Susceptible wild type mice (for example C57BI/6) show similiar symptoms if fed a high fat diet. In addition to testing the expression of the proteins of the invention in such mouse strains (see EXAMPLES section), these mice could be used to test whether administration of a candidate modulator alters for example lipid accumulation in the liver, in plasma, or adipose tissues using standard assays well known in the art, such as FPLC, colorimetric assays, blood glucose level tests, insulin tolerance tests and others.

Transgenic animals may be made through homologous recombination in embryonic stem cells, where the normal locus of the gene encoding the protein of the invention is mutated. Alternatively, a nucleic acid construct encoding the protein is injected into oocytes and is randomly integrated into the genome. One may also express the genes of the invention or variants thereof in tissues where they are not normally expressed or at abnormal times of development. Furthermore, variants of the genes of the invention like specific constructs expressing anti-sense molecules or expression of dominant negative mutations, which will block or alter the expression of the proteins of the invention may be randomly integrated into the genome. A detectable marker, such as lac Z or luciferase may be introduced into the locus of the genes of the invention, where upregulation of expression of the genes of the invention will result in an easily detectable change in phenotype. Vectors for stable integration include plasmids, retroviruses and other animal viruses, yeast artificial chromosomes (YACs), and the like. DNA constructs for homologous recombination will contain at least portions of the genes of the invention with the desired genetic modification, and will include regions of homology to the target locus. Conveniently, markers for positive and negative selection are included. DNA constructs for random integration do not need to contain regions of homology to mediate recombination. DNA constructs for random integration will consist of the nucleic acids encoding the proteins of the invention, a regulatory element (promoter), an intron and a poly-adenylation signal. Methods for generating cells having targeted gene modifications through homologous recombination are known in the field.

For embryonic stem (ES) cells, an ES cell line may be employed, or embryonic cells may be obtained freshly from a host, e.g. mouse, rat, guinea pig, etc. Such cells are grown on an appropriate fibroblast-feeder layer and are grown in the presence of leukemia inhibiting factor (LIF). ES or embryonic cells may be transfected and can then be used to produce transgenic animals. After transfection, the ES cells are plated onto a feeder layer in an appropriate medium. Cells containing the construct may be selected by employing a selection medium. After sufficient time for colonies to grow, they are picked and analyzed for the occurrence of homologous recombination. Colonies that are positive may then be used for embryo manipulation and morula aggregation. Briefly, morulae are obtained from 4 to 6 week old superovulated females, the Zona Pellucida is removed and the morulae are put into small depressions of a tissue culture dish. The ES cells are trypsinized, and the modified cells are placed into the depression closely to the morulae. On the following day the aggregates are transfered into the uterine horns of pseudopregnant females. Females are then allowed to go to term. Chimeric offsprings can be readily detected by a change in coat color and are subsequently screened for the transmission of the mutation into the next generation (F1-generation). Offspring of the F1-generation are screened for the presence of the modified gene and males and females having the modification are mated to produce homozygous progeny. If the gene alterations cause lethality at some point in development, tissues or organs can be maintained as allogenic or congenic grafts or transplants, or in vitro culture. The transgenic animals may be any non-human mammal, such as laboratory animal, domestic animals, etc., for example, mouse, rat, guinea pig, sheep, cow, pig, and others. The transgenic animals may be used in functional studies, drug screening, and other applications and are useful in the study of the function and regulation of the proteins of the invention in vivo.

The Figures show:
Figure 1 shows the triglyceride content of a spermidine synthase (GadFly Accession Number CG8327) mutant. Shown is the increase of triglyceride content of EP(3)3054 flies (referred to as 'EP(3)3054') caused by homozygous viable integration of the P-vector in comparison to controls with integration of this vector type elsewhere in the genome (referred to as 'EP-control').
Figure 2 shows the molecular organisation of the mutated spermidine synthase (Gadfly Accession Number CG8327) gene locus.
Figure 3 shows the mammalian homologs of GadFly Accession Number CG8327.
Figure 3A shows the BLASTP search result for the CG8327 gene product (Query) with the best human homolog match (Sbject).
Figure 3B shows the nucleic acid sequence of human spermidine synthase (SEQ ID NO: 1; GenBank Accession Number NM_003132).
Figure 3C shows the amino acid sequence (one-letter code) of human spermidine synthase (SEQ ID NO: 2; GenBank Accession Number NP_003123).
Figure 3D shows the Clustal X (1.81) protein sequence alignment for human spermidine synthase (referred to as 'Hs NP_003123'), murine spermidine synthase (referred to as 'Mm NP_033298'), and Drosophila spermidine synthase (referred to as 'Dm CG8327'). Gaps in the alignment are represented as -.
Figure 4 shows the expression of spermidine synthase in mammalian tissues. The relative RNA-expression is shown on the Y-axis. In Figure 4A and B, the tissues tested are given on the X-axis. "WAT" refers to white adipose tissue, "BAT" refers to brown adipose tissue. In Figure 4C and D, the X-axis represents the time axis. "d0" refers to day 0 (start of the experiment), "d2" - "d10" refers to day 2 - day 10 of adipocyte differentiation.
Figure 4A: Real-time PCR analysis of spermidine synthase expression in wildtype mouse tissues.
Figure 4B: Real-time PCR mediated analysis of spermidine synthase expression in different mouse models.
Figure 4C: Real-time PCR mediated analysis of spermidine synthase expression during the differentiation of 3T3-L1 cells from preadipocytes to mature adipocytes.
Figure 4D: Real-time PCR mediated analysis of spermidine synthase expression during the differentiation of 3T3-F442A cells from preadipocytes to mature adipocytes.

The examples illustrate the invention:

### Example 1: Measurement of triglyceride content

Mutant flies are obtained from a proprietary fly mutation stock collection and a publicly availabla stock collection. The flies are grown under standard conditions known to those skilled in the art. In the course of the experiment, additional feedings with bakers yeast (Saccharomyces cerevisiae) are provided. The average increase of triglyceride content of Drosophila containing the EP-vectors in homozygous or hemizygous viable integration was investigated in comparison to control flies (see Figure 1). For determination of triglyceride, flies were incubated for 5 min at 90°C in an aqueous buffer using a waterbath, followed by hot extraction. After another 5 min incubation at 90°C and mild centrifugation, the triglyceride content of the flies extract was determined using Sigma Triglyceride (INT 336-10 or -20) assay by measuring changes in the optical density according to the manufacturer's protocol. As a reference protein content of the same extract was measured using BIO-RAD DC Protein Assay according to the manufacturer's protocol. The assay was repeated three times.

The average triglyceride level of all flies of the EP collections (referred to as 'EP-control') is shown as 100% in the first columns in Figure 1, including standard deviation.

EP(3)3054 homozygous flies show constantly a higher triglyceride content than the control flies tested (column 2 in Figure 1). Therefore, the loss of gene activity in the locus 85E4 on chromosome 3R where the EP-vector of EP(3)3054 flies is homozygous viable integrated, is responsible for changes in the metabolism of the energy storage triglycerides.

### Example 2: Identification of Drosophila genes associated with metabolic control

The nucleic acids encoding the protein of the present invention was identified using a plasmid-rescue technique. Genomic DNA sequence was isolated that is localized adjacent to the EP vector (herein EP(3)3054) integration. Using this isolated genomic sequence public databases like Berkeley Drosophila Genome Project (GadFly) were screened thereby identifying the integration sites of the vector, and the corresponding gene. The molecular organization of this gene locus is shown in Figure 2.

In Figure 2, genomic DNA sequence is represented by the assembly as a scaled black line in the middle (numbers represent the length in basepairs of the genomic DNA) that includes the integration site of vector for line EP(3)3054. A transcribed DNA sequence (EST) and predicted genes are shown as bars on the upper side (sense strand). Predicted exons of the cDNA with GadFly Accession Number CG8327 are shown as dark grey bars and introns as light grey lines. The sequence encodes for a gene that is predicted by GadFly sequence analysis programs as Accession Number CG8327. Public DNA sequence databases (for example, NCBI GenBank) were screened thereby identifying the integration site of line EP(3)3054, causing an increase of triglyceride content. EP(3)3054 is integrated into the first cDNA in antisense orientation of the cDNA with Accession Number CG8327 (the site of integration is shown as triangle in the lower half of the Figure). Therefore, expression of the cDNA encoding Accession Number CG8327 could be effected by homozygous viable integration of vectors of line EP(3)3054, leading to increase of the energy storage triglycerides.

### Example 3: Identification of human homologous genes and proteins

The Drosophila gene and protein encoded thereby with functions in the regulation of triglyceride metabolism were further analysed using the BLAST algorithm searching in publicly available sequence databases and mammalian homologs were identified (see Figure 3).

As shown in Figure 3A, the gene product of Drosophila CG8327 is 76% homologous over 283 amino acids (of 302 amino acids) to a human spermidine synthase (also referred to as spermidine synthase-1; SRM; GenBank Accession Number NM_003132 for the cDNA, NP_003123 for the protein). Furthermore the gene product of Drosophila CG8327 is 76% homologous over 283 amino acids (of 302 amino acids) to murine spermidine synthase (GenBank Accession Number NM_009272 for the cDNA, NP_033298 for the protein). In Figure 3B and 3C, the nucleic acid and amino acid sequences of the human spermidine synthase are shown. Figure 3D shows a Clustal X (1.81) protein alignment of spermidine synthase homologs of human, mouse, and Drosophila.

### Example 4: Expression profiling experiments

To analyze the expression of the polypeptides disclosed in this invention in mammalian tissues, several mouse strains (preferrably mouse strains C57BI/6J, C57BI/6 ob/ob and C57BI/KS db/db which are standard model systems in obesity and diabetes research) were purchased from Harlan Winkelmann (33178 Borchen, Germany) and maintained under constant temperature (preferrably 22°C), 40 per cent humidity and a light / dark cycle of preferrably 14 / 10 hours. The mice were fed a standard diet (for example, from ssniff Spezialitäten GmbH, order number ssniff M-Z V1126-000). For the fasting experiment ("fasted-mice"), wild type mice were starved for 48 h without food, but only water supplied ad libitum (see, for example, Schnetzler et al. J Clin Invest 1993 Jul;92(1):272-80, Mizuno et al. Proc Natl Acad Sci U S A 1996 Apr 16;93(8):3434-8). Animals were sacrificed at an age of 6 to 8 weeks. The animal tissues were isolated according to standard procedures known to those skilled in the art, snap frozen in liquid nitrogen and stored at -80°C until needed.

For analyzing the role of the proteins disclosed in this invention in the in vitro differentiation of different mammalian cell culture cells for the conversion of pre-adipocytes to adipocytes, mammalian fibroblast (3T3-L1) cells (e.g., Green & Kehinde, Cell 1: 113-116, 1974) were obtained from the American Tissue Culture Collection (ATCC, Hanassas, VA, USA; ATCC- CL 173). 3T3-L1 cells were maintained as fibroblasts and differentiated into adipocytes as described in the prior art (e.g., Qiu. et al., J. Biol. Chem. 276:11988-95, 2001; Slieker et al., BBRC 251: 225-9, 1998). At various time points of the differentiation procedure, beginning with day 0 (day of confluence) and day 2 (hormone addition; for example, dexamethasone and 3-isobutyl-1-methylxanthine), up to 10 days of differentiation, suitable aliquots of cells were taken every two days. Alternatively, mammalian fibroblast 3T3-F442A cells (e.g., Green & Kehinde, Cell 7: 105-113, 1976) were obtained from the Harvard Medical School, Department of Cell Biology (Boston, MA, USA). 3T3-F442A cells were maintained as fibroblasts and differentiated into adipocytes as described previously (Djian, P. et al., J. Cell. Physiol., 124:554-556, 1985). At various time points of the differentiation procedure, beginning with day 0 (day of confluence and hormone addition, for example, Insulin), up to 10 days of differentiation, suitable aliquots of cells were taken every two days. 3T3-F442A cells are differentiating in vitro already in the confluent stage after hormone (insulin) addition.

RNA was isolated from mouse tissues or cell culture cells using Trizol Reagent (for example, from Invitrogen, Karlsruhe, Germany) and further purified with the RNeasy Kit (for example, from Qiagen, Germany) in combination with an DNase-treatment according to the instructions of the manufacturers and as known to those skilled in the art. Total RNA was reverse transcribed (preferrably using Superscript II RNaseH- Reverse Transcriptase, from Invitrogen, Karlsruhe, Germany) and subjected to Taqman analysis preferrably using the Taqman 2xPCR Master Mix (from Applied Biosystems, Weiterstadt, Germany; the Mix contains according to the Manufacturer for example AmpliTaq Gold DNA Polymerase, AmpErase UNG, dNTPs with dUTP, passive reference Rox and optimized buffer components) on a GeneAmp 5700 Sequence Detection System (from Applied Biosystems, Weiterstadt, Germany).

Taqman analysis of spermidine synthase (Srm) was performed preferrably using the following primer/probe pairs: mouse Srm forward primer (Seq ID NO: 28) 5'- CCG TGC CGC CTT CGT AC -3'; mouse Srm reverse primer (Seq ID NO: 29) 5'- ACC TGG ATT CAG CTT ATG TCA TTG -3'; mouse Srm Taqman probe (Seq ID NO: 30) (5/6-FAM) CCT GAG TTC ACC CGG AAG GCC C (5/6-TAMRA).

Real time PCR (Taqman) analysis of the expression of spermidine synthase in mammalian (mouse) tissues revealed that spermidine synthase is expressed in a variety of adult mouse tissues including WAT and BAT. (Figure 4A). The high experession levels of spermidine synthase in these tissues indicate, that spermidine synthase is involved in the metabolism of tissues relevant for the metabolic syndrome. The expression of spermidine synthase in brown adipose tissue is under metabolic control: In genetically obese (ob/ob) mice, expression is moderately induced compared to wildtype levels (Figure 4B). In addition, expression of spermidine synthase is regulated during the in vitro differentiation of 3T3-L1 cells as well as of an additional model system for the in vitro differentiation of preadipocytes to adipocytes, the 3T3-F442A cell line (Figures 4C and 4D)., Whereas spermidine synthase shows a transient upregulation of its expression in3T3-L1 cells, a stable upregulation of its expression in 3T3-F442A cells is noted. Spermidine synthase is upregulated by the end of the clonal expansion phase of preadipocyte differentaition. This suggests that spermidine synthase is necessary during the onset of final adipocyte maturation (see Figures 4C and 4D).

## Claims

1. Use of a nucleic acid molecule of human spermidine synthase (SRM) gene or a polypeptide encoded thereby or an antibody, or an aptamer recognizing a nucleic acid molecule of the SRM gene or a polypeptide encoded thereby for the manufacture of a medicament for the treatment, alleviation and/or prevention of obesity and related disorders, selected from obesity, body weight regulation disorders, eating disorder, cachexia, diabetes mellitus, hypertension, coronary heart disease, hypercholesterolemia and dyslipidemia, wherein the nucleic acid molecule of the SRM gene is
(i) encoding Drosophila Gadfly Accession Number CG8327, human spermidine synthase (SRM; SEQ ID NO: 1; GenBank Accession Number NM_003132) and/or a sequence which is complementary thereto, or
(ii) a nucleic acid molecule that
(a) hybridizes at 50°C in a solution containing 1 x SSC and 0.1% SDS to a nucleic acid molecule as defined in (i) or a nucleic acid molecule which is complementary thereto; or
(b) is degenerate with respect to the nucleic acid molecule of (a); or
(c) encodes a polypeptide which is at least 85%, preferably at least 90%, more preferably at least 95%, more preferably at least 98% and up to 99,6% identical to a human spermidine synthase (SRM; SEQ ID NO: 2; GenBank Accession Number NP_003123) as defined in (i); or
(d) differs from the nucleic acid molecule of (a) to (c) by mutation and wherein said mutation causes an alteration, deletion, duplication or premature stop in the encoded polypeptide.

2. The use of claim 1, wherein the medicament further comprises pharmaceutically acceptable carriers, diluents and/or adjuvants.

3. The use of claim 1 or 2, wherein the nucleic acid molecule is a DNA molecule, particularly a cDNA or a genomic DNA.

4. The use of any one of claims 1-3, wherein said nucleic acid encodes a polypeptide contributing to regulating the energy homeostasis and/or the metabolism of triglycerides.

5. The use of any one of claims 1-4, wherein said nucleic acid molecule is a recombinant nucleic acid molecule.

6. The use of any one of claims 1-5, wherein the nucleic acid molecule is a vector, particularly an expression vector.

7. The use of any one of claims 1-4, wherein the polypeptide is a recombinant polypeptide.

8. The use of claim 7, wherein said recombinant polypeptide is a fusion polypeptide.

9. The use of any one of claims 1-6. wherein said nucleic acid molecule is selected from anti-sense oligonucleotides.

## Patentansprüche

1. Verwendung eines Nukleinsäuremoleküls eines Gens oder eines davon kodierten Polypeptids einer humanen Spermidinsynthase (SRM) oder eines Antikörpers oder eines Aptamers, welcher bzw. welches ein Nukleinsäuremolekül des SRM-Gens oder ein davon kodiertes Polypeptid erkennt, zur Herstellung eines Medikamentes für die Behandlung, Linderung und/oder Vorbeugung von Fettleibigkeit und verwandten Störungen, ausgewählt aus Fettleibigkeit, Störungen der Regulierung des Körpergewichtes, Essstörung, Kachexie, Diabetes mellitus, Hypertonie, koronare Herzerkrankung, Hypercholesterinämie und Dyslipidämie, wobei das Nukleinsäuremolekül des SRM-Gens
(i) Drosophila Gadfly Zugangsnummer CG8327, humane Spermidinsynthase (SRM; SEQ ID Nr: 1; GenBank Zugangsnummer NM_003132) und/oder eine Sequenz kodiert, die dazu komplementär ist, oder
(ii) ein Nukleinsäuremolekül ist, welches
(a) bei 50°C in einer Lösung, die 1 x SSC und 0,1% SDS enthält, an ein wie in (i) definiertes Nukleinsäuremolekül oder an ein dazu komplementäre Nukleinsäuremolekül hybridisiert, oder
(b) in Bezug auf das Nukleinsäuremolekül von (a) degeneriert ist, oder
(c) ein Polypeptid kodiert, das zu mindestens 85%, vorzugsweise zu mindestens 90%, mehr bevorzugt zu mindestens 95%, mehr bevorzugt zu mindestens 98% und bis zu 99,6% identisch ist zu einer wie in
(i) definierten humanen Spermidinsynthase (SRM; SEQ ID Nr: 2; GenBank Zugangsnummer NM_003123), oder
(d) sich von dem Nukleinsäuremolekül von (a) bis (c) durch Mutation unterscheidet und wobei die Mutation eine Veränderung, Deletion, Duplikation oder einen vorzeitigen Abbruch des kodierten Polypeptids verursacht.

2. Verwendung nach Anspruch 1, wobei das Medikament des Weiteren pharmazeutisch zulässige Trägerstoffe, Verdünnungsmittel und/oder Adjuvanzien umfasst.

3. Verwendung nach Anspruch 1 oder 2, wobei es sich bei dem Nukleinsäuremolekül um ein DNA-Molekül handelt, insbesondere um eine cDNA oder eine genomische DNA.

4. Verwendung nach einem der Ansprüche 1-3, wobei die Nukleinsäure ein Polypeptid kodiert, das zur Regulierung der Energiehomöostase und/oder des Triglyzeridstoffwechsels beiträgt.

5. Verwendung nach einem der Ansprüche 1-4, wobei es sich bei dem Nukleinsäuremolekül um ein rekombinantes Nukleinsäuremolekül handelt.

6. Verwendung nach einem der Ansprüche 1-5, wobei es sich bei dem Nukleinsäuremolekül um einen Vektor handelt, insbesondere um einen Expressionsvektor.

7. Verwendung nach einem der Ansprüche 1-4, wobei es sich bei dem Polypeptid um ein rekombinantes Polypeptid handelt.

8. Verwendung nach Anspruch 7, wobei es sich bei dem rekombinanten Polypeptid um ein Fusionspolypeptid handelt.

9. Verwendung nach einem der Ansprüche 1-6, wobei das Nukleinsäuremolekül aus Antisense-Oligonukleotiden ausgewählt ist.

## Revendications

1. Utilisation d'une molécule d'acide nucléique du gène de la spermidine synthase humaine (SRM) ou d'un polypeptide codé par celle-ci ou un d'anticorps, ou d'un aptamère reconnaissant une molécule d'acide nucléique du gène de la SRM ou d'un polypeptide codé par celle-ci pour la fabrication d'un médicament destiné au traitement, au soulagement et/ou à la prévention de l'obésité et des troubles apparentés, choisis parmi l'obésité, les troubles de régulation du poids corporel, les troubles de l'alimentation, la cachexie, le diabète sucré, l'hypertension, une maladie coronarienne, l'hypercholestérolémie et la dyslipidémie, dans laquelle la molécule d'acide nucléique du gène de la SRM
(i) code pour le numéro d'accès CG8327 de la base de données Gadfly sur la drosophile, spermidine synthase humaine (SRM ; SEQ ID N° : 1 ; numéro d'accès GenBank NM_003132) et/ou une séquence qui est complémentaire de celle-ci, ou
(ii) est une molécule d'acide nucléique qui
(a) s'hybride à 50°C dans une solution contenant du SSC 1x et du SDS à 0,1 % à une molécule d'acide nucléique telle que définie dans (i) ou à une molécule d'acide nucléique qui est complémentaire de celle-ci ; ou
(b) est dégénérée relativement à la molécule d'acide nucléique de (a) ; ou
(c) code pour un polypeptide qui est identique à au moins 85 %, de préférence au moins 90 %, de manière davantage préférée au moins 95 %, de manière davantage préférée au moins 98 % et jusqu'à 99,6 % à la spermidine synthase humaine (SRM ; SEQ ID N° : 2 ; numéro d'accès GenBank NP_003123) tel que défini dans (i) ; ou
(d) diffère de la molécule d'acide nucléique de (a) à (c) par une mutation et dans laquelle ladite mutation entraîne une altération, une délétion, une duplication ou une interruption prématurée dans le polypeptide codé.

2. Utilisation selon la revendication 1, dans laquelle le médicament comprend en outre des vecteurs, diluants et/ou adjuvants pharmaceutiquement acceptables.

3. Utilisation selon la revendication 1 ou 2, dans laquelle la molécule d'acide nucléique est une molécule d'ADN, particulièrement un ADNc ou un ADN génomique.

4. Utilisation selon l'une quelconque des revendications 1 à 3, dans laquelle ledit acide nucléique code pour un polypeptide contribuant à la régulation de l'homéostasie énergétique et/ou au métabolisme des triglycérides.

5. Utilisation selon l'une quelconque des revendications 1 à 4, dans laquelle ladite molécule d'acide nucléique est une molécule d'acide nucléique recombinante.

6. Utilisation selon l'une quelconque des revendications 1 à 5, dans laquelle la molécule d'acide nucléique est un vecteur, particulièrement un vecteur d'expression.

7. Utilisation selon l'une quelconque des revendications 1 à 4, dans laquelle le polypeptide est un polypeptide recombinant.

8. Utilisation selon la revendication 7, dans laquelle ledit polypeptide recombinant est un polypeptide de fusion.

9. Utilisation selon l'une quelconque des revendications 1 à 6, dans laquelle ladite molécule d'acide nucléique est choisie parmi des oligonucléotides antisens.
